# EUROPEAN PATENT SPECIFICATION

(11) **EP 4 183 309 B1**
(45) Date of publication and mention of the grant of the patent: **18.03.2026**
(21) Application number: 21209850.3
(22) Date of filing: 23.11.2021
(51) Int. Cl.: A61B 1/00

(54) **ENDOSCOPE COMPRISING GEARED ELEVATOR MECHANICS**
ENDOSKOP MIT VERZAHNTEM ANHEBEMECHANISMUS
ENDOSCOPE COMPRENANT UNE MÉCANIQUE D'ÉLÉVATEUR À ENGRENAGES

(43) Date of publication of application: 24.05.2023
(73) Proprietor: Ambu A/S, 2750 Ballerup (DK)
(72) Inventor: TEATINI, Andrea, 86916 Kaufering (DE); MANN, Thomas, 86343 Königsbrunn (DE); DOHERTY, Kevin, 81543 München (DE); DO, Anh Minh, 81243 München (DE)
(74) Representative: Winter, Brandl - Partnerschaft mbB

(56) References cited:
- CN-A- 111 012 287
- JP-A- 2014 128 465
- US-A1- 2011 077 461

## Description

The present disclosure relates to an endoscope, in particular duodenoscope, comprising: an endoscope handle comprising a handle housing and at least one manually operable element; and an insertion cord configured to be inserted into a patient's body cavity and comprising a distal tip unit, the distal tip unit comprising an elevator element movable between a first lowered elevator position and a second raised elevator position for changing a direction of a tool or an instrument inserted into the patient's body cavity via a working channel of the endoscope; wherein the manually operable element is connected to the elevator element via elevator mechanics provided inside the handle housing and a wire, and a manual operation of the manually operable element is transmitted via the elevator mechanics and the wire to the elevator element such that a first operation position of the manually operable element corresponds to the first lowered elevator position and a second operation position of the manually operable element corresponds to the second raised elevator position.

### Related art

Endoscopes and similar specialized instruments such as bronchoscopes, arthroscopes, colonoscopes, laparoscopes, gastroscopes and duodenoscopes are well known from the state of the art and are used for visual examination and diagnosis of hollow organs and body cavities, as well as to assist in surgery, e.g. for a targeted tissue sampling. Basically, a distal tip unit of an endoscope, which is usually connected to a proximal endoscope handle via a bending section and an insertion tube, can be inserted into a hollow organ or body cavity to be investigated with the endoscope. Both reusable and disposable, i.e. single-use, endoscopes are known from the state of the art.

Some endoscopes, in particular duodenoscopes, comprise a distal tip unit having a shovel-like elevator element, a so-called Albarran lever, via which a direction of a tool or an instrument inserted into a patient's body cavity via a working channel of the endoscope can be changed. The elevator element can be raised and lowered in order to set an angle with respect to a longitudinal axis of the distal tip unit at which the tool or instrument exits the distal tip unit into the patient's body cavity. In a raised position of the elevator element, the tool or instrument may e.g. point in a direction essentially perpendicular to the longitudinal axis of the distal tip unit. It is known to control the elevator element via a manually operable element like a control lever provided at the endoscope handle. Elevator mechanics are usually provided via which the manually operable element is connected to a (pull) wire which extends through the insertion cord of the endoscope and which is connected to the elevator element in the distal tip unit.

In related art endoscopes, it is known to invert a motion of the manually operable element by the elevator mechanics such that a downward-movement of the manually operable element results in an upward-movement of the elevator element and that an upward-movement of the manually operable element results in a downward-movement of the elevator element. An operator of related art endoscopes is used to said described motion inversion.

In order to reach the motion inversion it is known from related art endoscopes to connect a slider rod to an operation wheel connected to the manually operable element at a portion of the operation wheel which is essentially/ approximately diametrically opposed to a portion of the operation wheel where the manually operable element is connected to the operation wheel. Said differently, the slider rod is not connected to the operation wheel at a side of the same where the manually operable element is provided, but is connected to the operation wheel at an opposed side. Therefore, the slider rod moves around inside a handle housing of the endoscope handle, in particular in a central portion of an accommodation space defined by the handle housing or even in a portion of the handle housing which is close to a surface/ wall which is opposed to a surface/ wall at which the manually operable element is provided.

Due to the moving slider rod inside the handle housing, much installation/ accommodation space provided inside the handle housing cannot be used for other parts/ components like valves, which are intended to be accommodated inside the handle housing.

US 5 507 717 A e.g. discloses an endoscope with an elevator at its distal end, wherein the elevator is controlled by a control lever, which is connected to an elevator rod and a slider.

Further, US 5 460 168 A discloses an endoscope with an elevator at the distal tip. The elevator is controlled by a lever. The lever is connected to a control lever gear wheel. The control lever gear wheel has teeth that are in engaging contact with a toothed portion of a connecting member formed as a rack that is movable in an axial direction. The connecting member is connected to a wire that runs to the elevator at the distal tip. JP 3 349 804 B2 discloses an endoscope with an elevator, which is controlled by a lever. In an embodiment, the lever is connected to a larger gear wheel that is in meshing contact with a smaller gear wheel. A wire is sandwiched between the two gear wheels. A wire end is not connected to any component/ part.

Moreover, CN 111012287 A discloses a single-use endoscope with a forceps lifter at its distal tip. The lifter is activated by a lever. The lever is connected to a first gear wheel that meshes with a second gear wheel. The second gear wheel meshes with a third gear wheel. The third gear wheel is in contact with a rack that moves in an axial direction. The movement of the rack drives a wire that is connected to the forceps lifter. CN 111012287 discloses the preamble of independent claim 1.

The elevator mechanics provided in CN 111012287 A have the disadvantage that they need much installation space. Moreover, the plurality of meshing engagements provided between the plurality of gear wheels and the toothed rack leads an increased play in the elevator mechanics, which is also disadvantageous.

Further, JP 2014 128465 A discloses a manually operable element having a bevel gear engaging another bevel gear provided on a rotary shaft, which leads to a rotation of a plurality of components/ shafts. The rotation of a distal shaft of the plurality of components/ shafts is transmitted via a worm gear into a movement of an elevator element. JP 2014 128465 discloses the preamble of independent claim 1.

### Brief description of the disclosure

In view of the above-described problems, it is an object of the present disclosure to avoid or at least to mitigate the problems of the related art, in particular to provide an endoscope having an endoscope handle comprising a handle housing, which is compact and in which an available installation space is appropriately used, and comprising a manually operable element which is connected to an elevator element provided in the distal tip unit of the endoscope via simple elevator mechanics having in particular a low number of parts, wherein the elevator mechanics are configured such that when the manually operable element is moved in a downward or distal direction, the elevator element is moved to a raised elevator position, and when the manually operable element is moved in an upward or proximal direction, the elevator element is moved to a lowered elevator position.

This object is solved by an endoscope in accordance with claim 1 and by a system comprising an endoscope and a monitor in accordance with claim 14. Advantageous aspects of the present disclosure are claimed in the dependent claims and/or are described herein below.

The invention as defined in independent claim 1 relates to an endoscope comprising: an endoscope handle comprising a handle housing and at least one manually operable element; and an insertion cord configured to be inserted into a patient's body cavity and comprising a distal tip unit, the distal tip unit comprising an elevator element movable between a first lowered elevator position and a second raised elevator position for changing a direction of a tool or an instrument inserted into the patient's body cavity via a working channel of the endoscope; wherein the manually operable element is connected to the elevator element via elevator mechanics provided inside the handle housing and a pull wire, and a manual operation of the manually operable element is transmitted via the elevator mechanics and the pull wire to the elevator element such that a first operation position of the manually operable element corresponds to the first lowered elevator position and a second operation position of the manually operable element corresponds to the second raised elevator position; and wherein the elevator mechanics comprise: an operation wheel connected to the manually operable element and comprising an operation wheel gear portion; and a wire wheel comprising a wire wheel gear portion; wherein the operation wheel gear portion is in meshing engagement with the wire wheel gear portion; wherein the pull wire is connected to the wire wheel; and wherein the pull wire is directly attached to a portion of the wire wheel or is indirectly connected to the wire wheel via a connector part which is rotatably mounted on the wire wheel and to which the pull wire is fixedly attached.

Summarized, according to the present disclosure an endoscope is provided, which comprises an endoscope handle. The endoscope handle comprises a handle housing and a manually operable element. The endoscope further comprises an insertion cord comprising a distal tip unit. The distal tip unit comprises an elevator element, which is movable between a first elevator position and a second elevator position. The manually operable element is connected to the elevator element via (geared) elevator mechanics and a wire. A manual operation of the manually operable element is transmitted via the elevator mechanics and the wire to the elevator element such that a first operation position of the manually operable element corresponds to the first elevator position and a second operation position of the manually operable element corresponds to the second elevator position. The elevator mechanics comprise an operation wheel connected to the manually operable element and comprising an operation wheel gear portion. The elevator mechanics further comprise a wire wheel comprising a wire wheel gear portion. The operation wheel gear portion is in meshing engagement with the wire wheel gear portion. The wire is connected to the wire wheel.

The present disclosure thus provides geared elevator mechanics, which use/ comprise (exactly) two gear wheels (the operation wheel having the operation wheel gear portion and the wire wheel having the wire wheel gear portion) for inverting a motion of the manually operable element, instead of the slider rod and the slider as known from the related art. The elevator mechanics in accordance with the present disclosure comprise few parts and thus do not need much installation space inside the handle housing. In other words, the installation space inside the handle housing is appropriately used and the handle housing may be formed compactly. As the elevator mechanics according to the present disclosure do not provide a slider rod moving around in a proximal end portion of the handle housing, the available space can be used for other components/ parts like valves and in addition, a risk of affecting a tubing or control wires by such a moving slider rod is excluded. Diameters of the gear wheels may be suitably set so as to provide a desired gearing and to occupy little installation space. Torques and forces for raising the elevator element may be set by appropriately defining the diameters of the gear wheels, a lever arm defined by a distance of the manually operable element to a rotational center of the operation wheel and a lever arm defined by a connection point of the wire to the wire wheel. The provision of (only) two gear wheels makes it possible that a play of the elevator mechanics is not unnecessarily increased.

The endoscope according to the present disclosure is preferably a single-use endoscope, i.e. is intended and designed for single-use only.

Further, the endoscope according to the present disclosure is preferably a duodenoscope, i.e. is especially designed for examining the duodenum.

The endoscope handle may comprise a working channel access port, and the working channel may extend from the working channel access port of the endoscope handle to the distal tip unit of the insertion cord. Further, the insertion cord may comprise an insertion tube and an actively bendable bending section in addition to the distal tip unit. There may be provided at least one, preferably two, operation unit like a control wheel at the endoscope handle for actively bending the bending section in at least one bending plane i.e. two directions, preferably in two bending planes i.e. four directions. The operation unit may be connected to a shaft, which again may be connected to a wire drum, in which steering/ control wires for controlling a bending movement of the bending section run. It is to be understood however that the present disclosure is not limited to a flexible endoscope having an insertion cord with an actively bendable bending section. I.e. the endoscope according to the present disclosure may also be a stiff endoscope, an insertion cord of which cannot be actively bent.

The elevator element of the present disclosure is preferably a shovel-like elevator element, in particular a so-called Albarran lever. In the first lowered elevator position, the elevator element is preferably arranged such that the instrument or tool may be inserted into the patient's body cavity in a direction, which is preferably only slightly angled with respect to the longitudinal axis direction of the distal tip unit. There is thus preferably only a slight change of direction (or even no change of direction) of the instrument or tool, which is inserted into the patient's body cavity via the working channel when the elevator element is in the first lowered elevator position. In the second raised elevator position, the elevator element is preferably arranged/ angled such that the instrument or tool is inserted into the patient's body cavity in a direction essentially perpendicular to the longitudinal axis direction of the distal tip unit. The (pull) wire preferably extends from the endoscope handle to the distal tip unit, preferably via the insertion tube and the bending section, and may be fixed/ attached to the elevator element in the distal tip unit.

Preferably, the manually operable element, via which the elevator element may be actuated/ operated, is moved or rotated in a distal direction, which an operator of the endoscope may perceive as downward-movement, in order to transfer the manually operable element from the first operation position to the second operation position and thus the elevator element form the first lowered elevator position to the second raised elevator position. Further, preferred, the manually operable element is moved or rotated in a proximal direction, which an operator of the endoscope may perceive as upward-movement, in order to transfer the manually operable element from the second operation position to the first operation position and thus the elevator element from the second raised elevator position to the first lowered elevator position. It is to be understood that in the context of the present disclosure "proximal" means away from a patient, towards a user/ operator and "distal" means towards the patient, away from the user/ operator. Moreover, it is to be understood that the manually operable element may be moved/ rotated in any position between the first operation position and the second operation position in order to adjust an angle with respect to the longitudinal axis of the distal tip unit, in which the instrument or tool is inserted into the patient's body cavity.

The manually operable element is preferably a control lever. The control lever may have a gripping surface adapted to and provided for being actuated/ operated by the user/ operator. The gripping surface may e.g. be ribbed, corrugated, grooved, serrated, recessed, etc., in other words may have a haptic structure so that the user can haptically perceive the manually operable element without looking at the same. Preferably, the gripping surface may be (convexly or concavely) curved, and may e.g. be adapted to a finger like a thumb of the user/ operator.

Preferably, the manually operable element is fixedly connected to the operation wheel, so that the manually operable element and the operation wheel rotate integrally with each other. I.e. a rotational input provided on the manually operable element by the user/ operator preferably results directly in a rotation of the operation wheel due to the fixed connection between the manually operable element and the operation wheel.

Further preferred, the manually operable element extends through an opening, which is in particular formed as a slot-shaped opening, provided in the handle housing so as to be accessible by the user/ operator. The manually operable element may be movable/ displaceable/ rotatable along the (slot-shaped) opening. The opening may define the first operation position and the second operation position of the manually operable element. In particular, when the manually operable element hits a proximal-most end of the opening, the manually operable element may be in the first operation position, and when the manually operable element hits a distal-most end of the opening, the manually operable element may be in the second operation position.

Preferably, the operation wheel is rotatably mounted in the handle housing of the endoscope handle, e.g. on a (cylindrical) protrusion provided inside the handle housing, and may thus rotate around its rotational center.

The operation wheel gear portion of the operation wheel may be an external gear/ may comprise external teeth. Basically, the operation wheel gear portion may be provided/ may extend over an entire outer circumference of the operation wheel, which according to an embodiment may be disc-shaped, i.e. may have a round/ circular outer circumference. Alternatively, the operation wheel gear portion may be provided only in a subarea, in particular in an angular range smaller 180°, preferably between 45° and 180°, on the outer circumference of the operation wheel. In particular, the subarea or angular range in which the operation wheel gear portion having the external teeth/ gear is provided may be adjusted to an angular range in which the manually operable element may be rotated inside the (slot-shaped) opening provided in the handle housing of the endoscope handle. The operation wheel may in this light advantageously have a shape corresponding to a portion or part of a disc, comprising at least the rotational center and the operation wheel gear portion in a subarea/ angular range, with portions of a hypothetical/ imaginary full disc removed. E.g. the operation wheel, which preferably has a flat shape, may not be circular/ round but may only have a round/ circular portion and two, preferably straight, edges, which are in particular angled with respect to each other. According to said preferred embodiment, less assembly space is occupied by the operation wheel inside the handle housing of the endoscope handle.

Further, the wire wheel may be rotatably mounted in the handle housing of the endoscope handle, e.g. on a (cylindrical) protrusion provided inside the handle housing, and may thus rotate around its rotational center.

The wire wheel gear portion of the wire wheel may be an external gear/ may comprise external teeth. Basically, the wire wheel gear portion may be provided/ may extend over an entire outer circumference of the wire wheel, which according to an embodiment may be disc-shaped, i.e. may have a round/ circular outer circumference. Alternatively, the wire wheel gear portion may be provided only in a subarea, in particular in an angular range smaller 270°, preferably between 45° and 270°, on the outer circumference of the wire wheel. In particular, the subarea or angular range in which the wire wheel gear portion having the external teeth/ gear is provided may be adjusted to the angular range of the operation wheel gear portion of the operation wheel. The wire wheel may in this light advantageously have a shape corresponding to a portion or part of a disc, comprising at least the rotational center and the wire wheel gear portion in a subarea/ angular range, with portions of a hypothetical/ imaginary full disc removed. E.g. the wire wheel, which preferably has a flat shape, may not be circular/ round but may only have a round/ circular portion and two, preferably straight, edges, which are in particular angled with respect to each other. According to said preferred embodiment, less assembly space is occupied by the wire wheel inside the handle housing of the endoscope handle.

The wire wheel is preferably arranged distally with respect to the operation wheel inside the handle housing of the endoscope handle. Therefore, more installation space is available in a proximal part/ portion of the endoscope handle for other components/ parts like valves.

Preferably, the operation wheel and the wire wheel have a flat shape, in particular a flat disc-shape. A flat shape of both the operation wheel and the wire wheel has the advantage that not much assembly space is occupied by the operation wheel and the wire wheel.

The operation wheel may rotate around an axis defined by its rotational center which may correspond to an axis around which the operation unit, the shaft and the wire drum (for bending the bending section of the insertion cord) can be rotated. Said differently, the operation wheel may be arranged concentrically and coaxially to the operation unit, the shaft and the wire drum. This embodiment in combination with the flat shape of the operation wheel makes it possible to suitably use an assembly space in a proximal portion of the handle housing. E.g. the operation wheel may have an annular/ ring-shape and may be supported by an outer shell surface of a (hollow) cylindrical protrusion provided in the handle housing, wherein on the inner shell surface of the (hollow) cylindrical protrusion the operation unit(s), the shaft(s) and the wire drum(s) may be suitably supported.

According to a preferred embodiment, a radius of the operation wheel gear portion (a distance between the gear portion of the operation wheel having external teeth and the rotational center of the operation wheel) may be greater than a radius of the wire wheel gear portion (a distance between the gear portion of the wire wheel having external teeth and the rotational center of the wire wheel). Said differently, a diameter of the operation wheel may be greater than a diameter of the wire wheel. I.e. the large gear wheel (operation wheel) may in a way serve as a lever for the (power transmission) system, and the small gear wheel (wire wheel) may serve for inverting the motion. This embodiment has further the advantage that in particular the wire wheel does not occupy much assembly space. A radius of the wire wheel gear portion and a radial position on the wire wheel where the wire is connected to the wire wheel may be adjusted such that an appropriate gearing may be provided and that the force introduced into the operation wheel of the elevator mechanics via the manually operable element is suitably transmitted to the wire for actuating the elevator element. E.g. when the radius of the operation wheel gear portion is greater than the radius of the wire wheel gear portion, a torque applied on the wire wheel is smaller than a torque applied to the operation wheel. The torque applied to the operation wheel is dependent on the force applied by the user/ operator and the lever arm, which is rather big for the operation wheel since the manually operable element preferably extends to the outside of the handle housing in order to be accessible to the user/ operator. Although the torque applied on the wire wheel is reduced, the radial position where the wire is connected to the wire wheel and thus the lever arm may be set appropriately (smaller than the lever arm of the manually operable element) so that the force which is applied to the wire may e.g. correspond to the force which is applied to the wire in a related art endoscope. Therefore, the user/ operator may perceive the operation of the elevator element as he is accustomed in related art systems. Said differently, the actuation of the elevator element preferably does not feel unusual or unfamiliar to the user/ operator. In some instances it may be advantageous to provide a construction wherein the radius of the operation wheel gear portion is smaller than the radius of the wire wheel gear portion, e.g. to increase the torque applied on the wire wheel to increase the force on the wire. The most simple solution is to have meshing wheels of circular or semi-circular shape, but if considered advantageous meshing wheels of non-circular shape can be used, e.g. to tailor the motion or torque development over the range of motion.

Preferably, a wire pipe is provided, which is fixed to the handle housing of the endoscope handle, wherein the wire runs into the wire pipe. I.e. the wire is preferably guided into the wire pipe in order to prevent that the wire arbitrarily moves around inside the endoscope handle. The wire pipe may be fixed in position by gluing the same to the handle housing of the endoscope handle. The wire pipe preferably extends essentially in the proximal-distal direction so as to suitably guide the wire from the endoscope handle into the insertion cord, where it is further guided to the distal tip unit and connected to the elevator element.

According to the present disclosure the wire is connected to the wire wheel. According to a preferred embodiment the wire may be directly attached/ fixed to a (radial outer) portion of the wire wheel, e.g. by gluing, tying a knot, crimping, a screwed connection, a soldered connection, etc., potentially in combination with looping a portion of the wire through an opening, a passage, a channel, a wire accommodating portion like a groove, etc. provided in the wire wheel. A direct attachment/ connection of the wire to a portion of the wire wheel has the advantage that the number of components/ parts used for the transmission of the manual operation a user/ operator applies to the manually operable element to the elevator element remains quite small, going along with an easy assembly, reduced costs, a reduced required assembly space, etc. A small disadvantage of said embodiment may however be seen in the fact that the wire might move around excessively inside the handle housing, when the wire wheel rotates, the wire having in particular a movement component which is perpendicular to a proximal-distal direction, i.e. horizontally, so that the wire may extend/ be directed in a direction which is angled with respect to the orientation/ direction of the wire pipe when entering the wire pipe. This may lead to a friction acting on the wire, which is disadvantageous, and may also lead to a force applied to the wire pipe which may lead to a disconnection of the wire pipe from the handle housing or even to a kinking of the wire pipe.

Therefore, according to another preferred embodiment a connector element/ part is provided, which is rotatably mounted on the wire wheel, wherein the wire is fixed/ fixedly attached to the connector element. According to said embodiment, the wire is in a way indirectly connected to the wire wheel via the connector element. According to the present disclosure a connection between the wire and the wire wheel therefore is to be understood as a direct or indirect connection (via another part/ component), as long as a movement of the wire wheel directly results in a movement of the wire, which is the case when the wire is fixedly attached to the mentioned connector element/ part and the connector element/ part (at least a portion mounted to the wire wheel) moves in accordance with a movement of the wire wheel, i.e. in a circle (i.e. both in the proximal-distal direction and perpendicular to the same/ horizontally), wherein a radius of the circle is defined by a distance of a mounting position of the connector element/ part to the rotational center of the wire wheel.

The elevator mechanics preferably comprise only two gear wheels, namely the operation wheel and the wire wheel. Preferably, no further wheel having a gear portion is in meshing engagement with the operation wheel and/ or the wire wheel. I.e. there is e.g. not provided a third wheel having a gear portion which is in meshing engagement with (gear portions of) the operation wheel and/ or the wire wheel.

According to the embodiment, which provides the connector element/ part, the wire wheel may have a protruding, (hollow) cylindrically formed (bearing) portion, which is provided radially away from the rotational center of the wire wheel, in a radially outer portion of the wire wheel, in particular close to the outer circumference of the wire wheel. The connector element/ part may be rotatably mounted on said (bearing) portion of the wire wheel.

According to said preferred embodiment, the wire wheel may have the wire wheel gear portion, the rotational center defining a rotation axis around which the wire wheel is rotatable, and the protruding, (hollow) cylindrically formed (bearing) portion. The rotation axis of the wire wheel may be parallel to an axis of said (bearing) portion.

Preferably, a guide rail element/ part may be provided which is rotatably mounted in the handle housing of the endoscope handle, wherein the connector element/ part is slidingly accommodated in the guide rail element/ part. According to an embodiment the wire pipe is fixedly attached to the guide rail element, e.g. by gluing. Attaching the wire pipe to the guide rail element will reduce the risk of the wire kinking outside the wire pipe, and could also reduce friction in the construction as there will be a relatively short portion of the wire not being protected by the wire pipe.

More specifically the connector element/ part may comprise a (disc-shaped) connector mounting portion configured to be rotatably mounted on the wire wheel, in particular on the bearing portion of the wire wheel, and a (rod-like) slide portion. The guide rail element/ part may comprise a rail mounting portion configured to be rotatably mounted on the handle housing and a guide rail portion (having a guide rail). The slide portion of the connector element may be slidingly accommodated in the guide rail portion of the guide rail element/ part. The rail mounting portion of the guide rail element/ part may be a distal portion of the guide rail element/ part and the guide rail portion may extend from the rail mounting portion in a proximal direction towards the wire wheel, in particular to the (bearing) portion of the wire wheel. The wire may be fixed, in particular by gluing, crimping, a screwed connection, a soldered connection, etc., to a portion of the connector part/ element. Especially preferred the wire is looped through, i.e. forms a loop in, the connector mounting portion of the connector element/ part, is fixed to the connector element/ part, in particular to the connector mounting portion of the connector element/ part, and extends through the slide portion of the connector element/ part.

Although a provision of the connector element/ part and of the guide rail element/ part may increase a number of components, it is to be understood that these additional components are preferably arranged distally with respect to the wire wheel, i.e. in a portion of the endoscope handle where it is easier to provide additional installation space. Therefore, the preferred embodiment having the connector element/ part and the guide rail element/ part in an advantageous way does not have moving parts in a proximal-most portion of the endoscope handle, so the installation space in the proximal-most portion of the endoscope handle is available for valves, the operation unit(s), shaft(s), wire drum(s), brake(s), etc.

The connector element/ part is preferably a plastic component/ part, especially preferred manufactured in an injection molding process. The same applies preferably for the guide rail element/ part.

A proximal end portion of the wire pipe may be arranged adjacent/ close to a distal end portion of the guide rail element/ part. Since the rail mounting portion of the guide rail element/ part is preferably provided at the distal end portion of the guide rail element, in particular adjacent a wire outlet opening of the guide rail element/ part, the wire outlet opening remains at an essentially stationary/ fixed position throughout an operation range of the manually operable element. In other words, the wire outlet opening preferably only experiences a slight rotational displacement. The proximal end portion of the wire pipe is especially preferred arranged adjacent/ close to the wire outlet opening of the guide rail element/ part.

An extension direction of the proximal end portion of the wire pipe may essentially correspond to an axis of the (guide rail portion of the) guide rail element and thus to an axis of the (rod-like) slide portion of the connector element/ part, at least in any position of the manually operable element between the first operation position and the second operation position.

The wire preferably runs from the wire pipe directly into the connector element/ part where it is attached/ fixed. The guide rail element/ part preferably serves for suitably orienting the connector element/ part, so that the wire runs directly and straightly into the wire pipe.

The handle housing of the endoscope handle may comprise two half-shell parts attached, in particular glued, to each other.

In a mounted state of the handle housing, the handle housing may be defined so as to have a proximal handle housing portion and a distal handle housing portion, the proximal handle housing portion and the distal handle housing portion merging integrally into each other. The elevator mechanics, in particular the operation wheel and the wire wheel, are preferably arranged at least partially in the proximal handle housing portion. The proximal handle housing portion may thus form a receiving space for the elevator mechanics and may be defined, in particular when looking at a proximal end of the endoscope handle, so as to have a top surface/ wall (portion), a bottom surface/ wall (portion), a first side surface/ wall (portion), a second side surface/ wall (portion) and a front surface/ wall (portion), wherein the top surface is opposite the bottom surface, the first side surface is opposite the second side surface, and the front surface is connected to the top surface, the bottom surface, the first side surface and the second side surface, and wherein the manually operable element may be provided/ may be displaceable/ rotatable in a rounded transition area between the bottom surface and the front surface. The top surface is preferably connected to the first and second side surfaces. The bottom surface is preferably connected to the first and second side surfaces. The front surface may be defined as proximal-most surface/ wall of the handle housing of the endoscope handle. Preferably rounded transition areas are provided between all surfaces so that the endoscope handle receives a modern design and lies comfortably in a hand of the user/ operator.

The manually operable element is preferably close to the front surface and distant from the bottom surface in the first operation position which corresponds to the first lowered elevator position. The manually operable element is preferably close to the bottom surface and distant from the front surface in the second operation position which corresponds to the second raised elevator position.

Preferably, the wire wheel is rotatably mounted in the handle housing, in particular on the first side surface or the second side surface, at a portion on the first side surface or the second side surface which is closer to the bottom surface than to the top surface.

Further, the connector part/ element is preferably mounted on the wire wheel at a portion of the wire wheel which is between the rotational center of the wire wheel and the bottom surface of the proximal handle housing portion.

Moreover, the operation unit(s), in particular control wheel(s), which may be provided as further manually operable element(s), and which are preferably provided for actively bending a bending section of the insertion cord, may be provided on the first side surface or the second side surface of the proximal handle housing portion. When the operation unit(s) is/ are provided on one of the side surfaces and the manually operable element for operating the elevator element is provided in a transition area between the front surface and the bottom surface, a risk of accidentally affecting the operation unit(s) is reduced when the manually operable element (the control lever) is operated. Therefore, according to the present disclosure the operation unit(s) and the manually operable element are preferably arranged on different surfaces of the proximal handle housing portion, so as to not accidentally influence each other.

Preferably, the connector part/ element and the guide rail part/ element are at least partially arranged distally with respect to the proximal handle housing portion, i.e. in the distal handle housing portion.

According to a preferred embodiment a valve assembly comprising a gas/ water injection valve and a suction valve is provided. The valve assembly is preferably arranged adjacent the elevator mechanics, i.e. adjacent the operation wheel and the wire wheel in the handle housing. The valve assembly is preferably arranged in the proximal handle housing portion of the endoscope handle. Especially preferred the valve assembly is attached/ provided on the top surface of the proximal handle housing portion.

Each of the valves may comprise a valve cylinder extending into the handle housing towards the elevator mechanics (the operation wheel and the wire wheel). Each of the valves may in addition comprise a plurality of tube ports extending (radially) away from the valve cylinders.

To sum up/ Said in different words, the present disclosure relates to an endoscope, which is preferably a duodensocope, wherein the endoscope comprises a distal tip (unit) with an elevator (element) being movable from a lowered position to a raised positon via elevator mechanics (provided) at a proximal end of the endoscope, in particular the endoscope handle, wherein the elevator mechanics comprise an operation wheel/ a control lever gear wheel connected to a manually operable element/ a control lever and being in engaging contact with a (toothed) wire wheel, which in turn is connected to an elevator wire running through the endoscope to the distal tip (unit) and connected to the elevator element.

The present disclosure further relates to a system comprising an endoscope as described above and a monitor.

### Brief description of figures

The disclosure is explained in more detail below using preferred embodiments and referring to the accompanying figures.
- Fig. 1: shows a side view of elevator mechanics provided in an endoscope handle in a related art endoscope.
- Fig. 2: shows a functional principle of the elevator mechanics of the related art endoscope.
- Fig. 3: shows an explanatory view of a power transmission chain between a manually operable element provided in the endoscope handle and an elevator element provided in a distal tip unit in the related art endoscope.
- Fig. 4: shows a side view of elevator mechanics provided in an endoscope handle according to a first preferred embodiment of the present disclosure, in which a manually operable element is in a first operation position.
- Fig. 5: shows a side view of the elevator mechanics according to the first preferred embodiment of the present disclosure, in which the manually operable element is in a second operation position.
- Fig. 6: shows a side view of the elevator mechanics according to the first preferred embodiment of the present disclosure, with operational parts not forming part of the elevator mechanics being removed.
- Fig. 7: shows a perspective view of the elevator mechanics according to the first preferred embodiment.
- Fig. 8: shows a side view of elevator mechanics provided in an endoscope handle according to a second preferred embodiment of the present disclosure.
- Fig. 9: shows a perspective view of the elevator mechanics provided in the endoscope handle according to the second preferred embodiment of the present disclosure.
- Fig. 10: shows another perspective view of the elevator mechanics provided in the endoscope handle according to the second preferred embodiment of the present disclosure.
- Fig. 11: shows a perspective view of the elevator mechanics provided in the endoscope handle according to the second preferred embodiment of the present disclosure, in which a manually operable element is in a first operation position.
- Fig. 12: shows a perspective view of the elevator mechanics provided in the endoscope handle according to the second preferred embodiment of the present disclosure, in which the manually operable element is in a second operation position.
- Fig. 13: shows a perspective view of an endoscope according to the second preferred embodiment, comprising an endoscope handle and an insertion cord, with an elevator element provided in a distal tip unit of the insertion cord, wherein a manually operable element provided at the endoscope handle is in the first operation position.
- Fig. 14: shows a perspective view of the endoscope according to the second preferred embodiment, comprising the endoscope handle and the insertion cord, with the elevator element provided in the distal tip unit of the insertion cord, wherein the manually operable element provided at the endoscope handle is in the second operation position.
- Fig. 15: shows a side view of a distal tip unit of the endoscope according to the present disclosure with an elevator element in a lowered elevator position.
- Fig. 16: shows a side view of a distal tip unit of the endoscope according to the present disclosure with the elevator element in a raised elevator position.

The figures are schematic in nature and serve only to understand the disclosure. The features of the different embodiments can be interchanged among each other.

### Detailed description of preferred embodiments

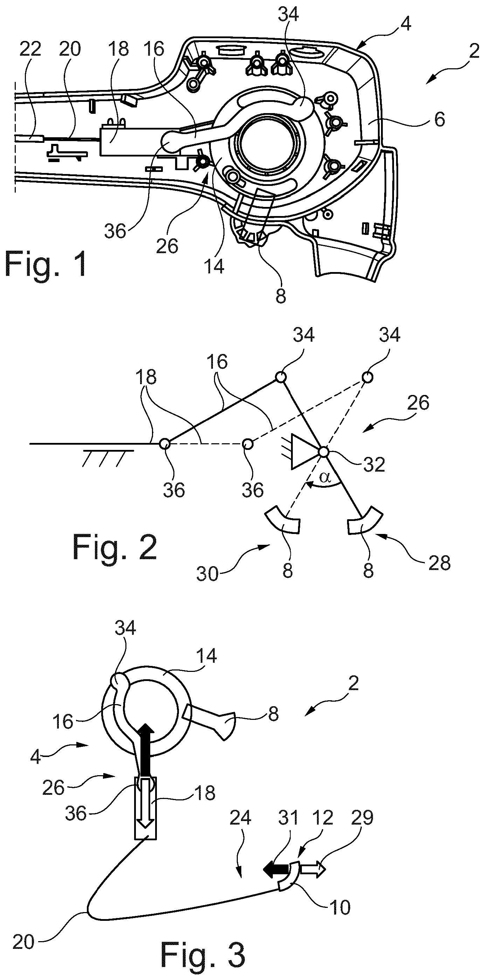

In Fig. 1, Fig. 2 and Fig. 3 a related art endoscope 2 is shown, which has an endoscope handle 4 having a handle housing 6 (only a half shell of the handle housing 6 is shown in Fig. 1) and a manually operable element 8 formed as a control lever, which can be rotated in order to move a shovel-like elevator element 10 (Albarran lever) provided in the distal tip unit 12 of the endoscope 2 between a lowered position and a raised position. A rotational input applied to the manually operable element 8 leads to a rotation of an operation wheel 14, which is connected to the manually operable element 8. A slider rod 16 is connected to the operation wheel 18 and is also connected to a slider 18. The slider 18 is slidingly arranged in the handle housing 6 of the endoscope handle 4. A wire 20 is fixed to the slider 18. The wire 20 runs into a wire pipe 22 attached to the handle housing 6 and further into an insertion cord 24 of the endoscope 2, in particular to the distal tip unit 12, where the wire 20 is fixed to the elevator element 10. A manual operation of the manually operable element 8 is thus transmitted via elevator mechanics 26 - comprising the operation wheel 14, the slider rod 16 and the slider 18 - and the wire 20 to the elevator element 10.

Fig. 2 schematically shows a functional principle of the elevator mechanics 26 of the related art endoscope 2. In solid lines a first operation position 28 of the manually operable element 8 is shown, which corresponds to a first lowered elevator position 29 of the elevator element 10 as seen in Fig. 3. In dashed lines a second operation position 30 of the manually operable element 8 is shown, which corresponds to a second raised elevator position 31 of the elevator element 10. Fig. 2 shows a rotational center 32 of the operation wheel 14, a first slider rod connection point 34, where the slider rod 16 is connected to the operation wheel 14, and a second slider rod connection point 36 where the slider rod 16 is connected to the slider 18. The manually operable element 8 may rotate around an angle α in order to move between the first operation position 28 and the second operation position 30. In particular, the manually operable element 8 is rotated in a distal direction, which an operator of the endoscope 2 perceives as downward-movement, in order to transfer the manually operable element 8 from the first operation position 28 to the second operation position 30 and thus the elevator element 10 form the first lowered elevator position 29 to the second raised elevator position 31. The manually operable element 8 is rotated in a proximal direction, which an operator of the endoscope 2 perceives as upward-movement, in order to transfer the manually operable element 8 from the second operation position 30 to the first operation position 28 and thus the elevator element 10 from the second raised elevator position 31 to the first lowered elevator position 29. Therefore, in the endoscope 2 a motion of the manually operable element 8 is basically inverted by the elevator mechanics 26 such that a downward-movement of the manually operable element 8 results in an upward movement of the elevator element 10 and that an upward-movement of the manually operable element 8 results in a downward-movement of the elevator element 10. This is even better illustrated in the explanatory view shown in Fig. 3. An operator of the endoscope 2 is used to said described motion inversion.

In order to reach the motion inversion in the related art endoscope 2 it is necessary to connect the slider rod 16 to the operation wheel 14 at a portion of the operation wheel 14 which is essentially/ approximately diametrically opposed to a portion of the operation wheel 14 where the manually operable element 8 is connected to the operation wheel 14. Said differently, the slider rod 16 may not be connected to the operation wheel 14 at a side of the same where the manually operable element 8 is provided, but has to be connected to the operation wheel 14 at an opposed side. Therefore, the slider rod 16 moves around inside the handle housing 6 of the endoscope handle, in particular in a central portion of an accommodation space defined by the handle housing 6 or even in a portion of the handle housing 6 which is close to a surface/ wall which is opposed to a surface/ wall at which the manually operable element 8 is provided.

Due to the moving slider rod 16 inside the handle housing 6, much installation/ accommodation space provided inside the handle housing 6 cannot be used for other parts/ components like valves which are intended to be accommodated inside the handle housing 6.

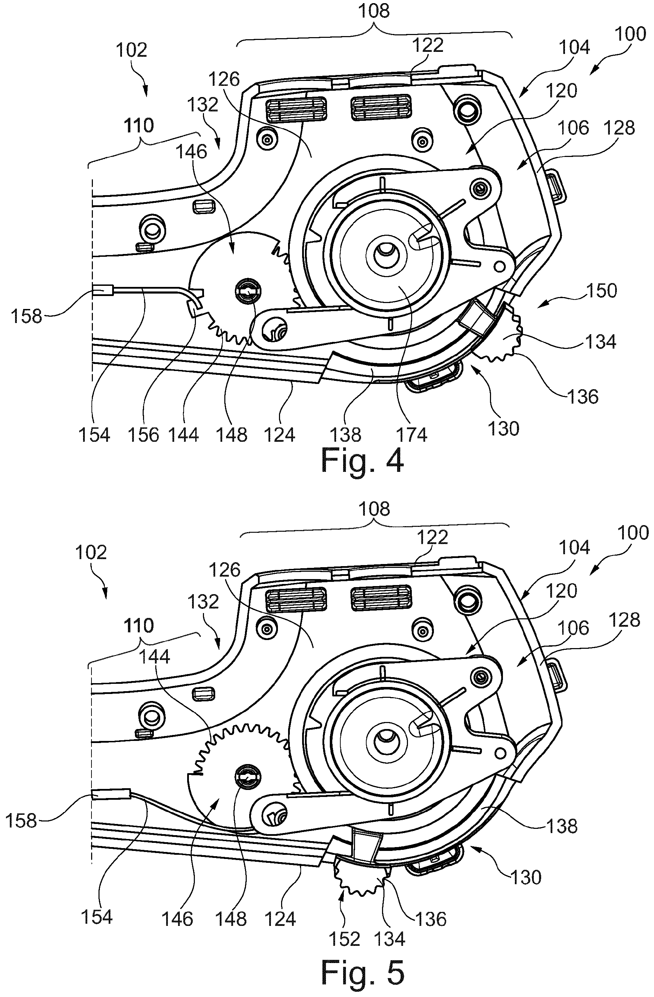

In Fig. 4, Fig. 5, Fig. 6 and Fig. 7 a first preferred embodiment of the present disclosure is shown. Fig. 4, Fig. 5, Fig. 6 and Fig. 7 show an endoscope handle 100 of an endoscope 102. The endoscope handle 100 comprises a handle housing 104. The handle housing 104 is formed by two half shells 106, which are attached to each other, e.g. by gluing. Only one of the half shells 106 is shown in the figures, with the other half shell 106 removed for showing parts/ components provided inside the handle housing 104. Fig. 4, Fig. 5, Fig. 6 and Fig. 7 each only show a proximal handle housing portion 108 in its entirety. A distal handle housing portion 110 which is integrally formed with the proximal handle housing portion 108 is only partially shown in the figures. Although not shown, it is evident that the endoscope 102 comprises an insertion cord 112 connected to the endoscope handle 100 and configured to be inserted into a patient's body cavity, wherein the insertion cord 112 comprises an insertion tube 114, an actively bendable bending section 116 and a distal tip unit 118.

The proximal handle housing portion 108 forms a receiving space 120 for components/ parts which are provided for operating the endoscope 102. In particular, the proximal handle housing portion 108 comprises a top surface/ wall 122, a bottom surface/ wall 124, first and second side surfaces/ walls 126 and a front surface/ wall 128. In the figures only one of the first and second side surfaces/ walls 126 is shown since one of the half shells 106 of the handle housing 104 has been removed. The top surface/ wall 122 is arranged opposite the bottom surface/ wall 124. The first and second side surfaces/ walls 126 are arranged opposite with respect to each other. The front surface/ wall 128 is connected to the top surface/ wall 122, the bottom surface/ wall 124 and the first and second side surfaces 126. A rounded transition area 130 is provided between the front surface/ wall 128 and the bottom surface/ wall 124. Preferably, the proximal handle housing portion 108 transitions to the distal handle housing portion 110 in a transition region 132 in which a cross-section of the handle housing 104 significantly changes.

In the rounded transition area 130 between the front surface/ wall 128 and the bottom surface/ wall 124 a manually operable element 134 formed as control lever is provided. The manually operable element 134 has a convexly curved, ribbed gripping surface 136, which is provided outside the handle housing 104 in order to be accessible to a user/ operator. The manually operable element 134 extends through a slot-shaped opening 138 provided in the handle housing 104 into the receiving space 120 defined by the proximal handle housing portion 108. The manually operable element 134 is fixedly connected to an operation wheel 140 provided inside the handle housing 104. The operation wheel 140 is rotatably mounted in the handle housing 104. A rotational input provided on the manually operable element 134 by the user/ operator results directly in a rotation of the operation wheel 140.

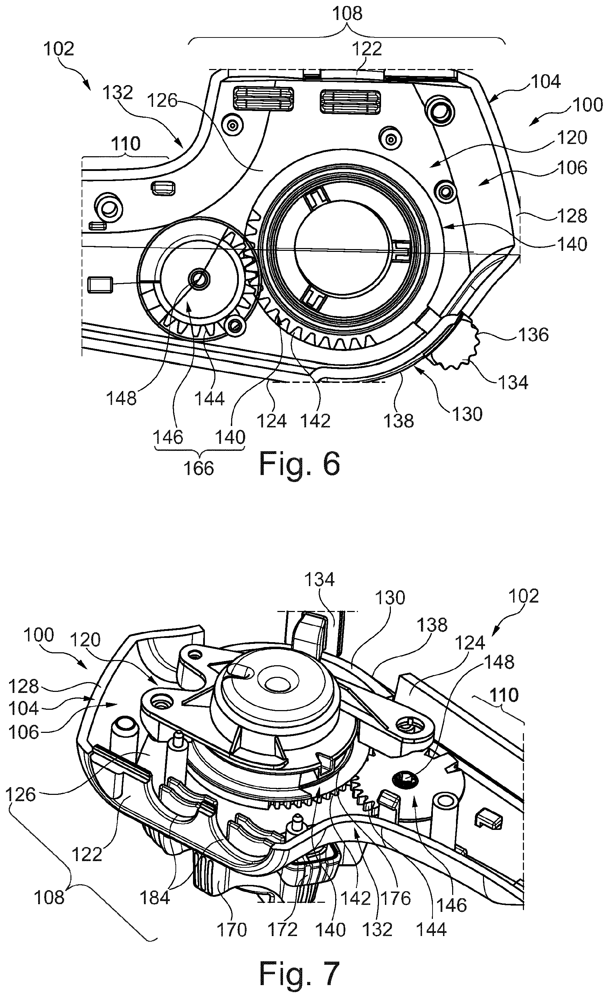

The operation wheel 140 comprises an operation wheel gear portion 142 having external teeth. As can be seen in particular in Fig. 6, the operation wheel 140 has an annular/ ring-like disc-shape. The operation wheel gear portion 142 having the external teeth is only provided in an angular range of around 130° to 140° on an outer circumference of the operation wheel 140. The operation wheel gear portion 142 of the operation wheel 140 is in meshing engagement with a wire wheel gear portion 144 of a wire wheel 146. Said differently the external teeth of the operation wheel gear portion 142 are in meshing engagement with external teeth of the wire wheel gear portion 144. The wire wheel 146 has essentially a disc-shape and is rotatably mounted in the handle housing 104, in particular around a cylindrical protrusion 148 provided in the handle housing 104. The wire wheel gear portion 144 having the external teeth is only provided in an angular range of around 180° to 190° on an outer circumference of the wire wheel 146. The wire wheel 146 is arranged distally with respect to the operation wheel 140, in particular partially inside the proximal handle housing portion 108 and inside the transition region 132 between the proximal handle housing portion 108 and the distal handle housing portion 110. A radius/ diameter of the operation wheel gear portion 142 is greater than a radius/ diameter of the wire wheel gear portion 144. As can be best seen in Fig. 7, the operation wheel 140 and the wire wheel 146 both have a flat shape.

The manually operable element 134 can be rotated between a first operation position 150 shown in Fig. 4 and a second operation position 152 shown in Fig. 5. The first operation position 150 and the second operation position 152 are defined by the slot-shaped opening 138, through which the manually operable element 134 extends. When the manually operable element 134 hits a proximal-most end of the slot-shaped opening 138 (close to the front surface/ wall 128), the manually operable element 138 is in the first operation position 150, and when the manually operable element 134 hits a distal-most end of the slot-shaped opening 138 (close to the bottom surface/ wall 124), the manually operable element 134 is in the second operation position 152.

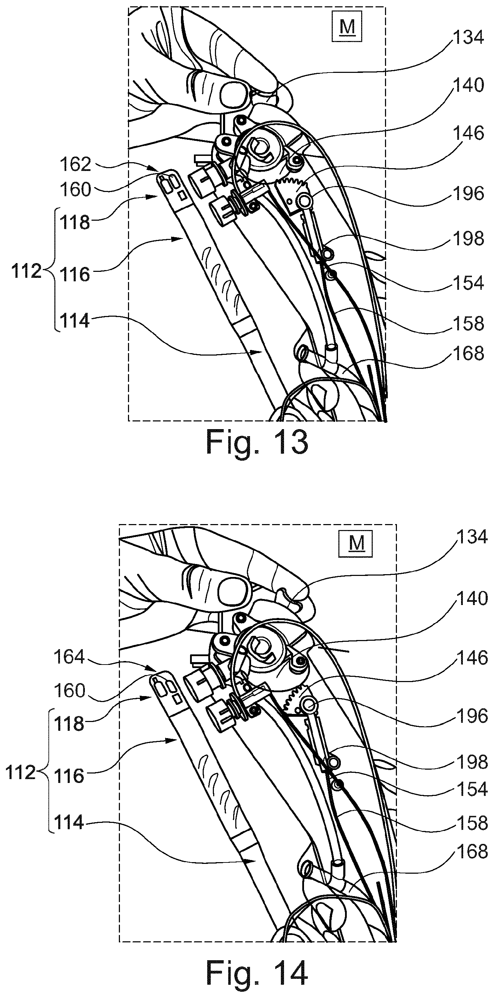

As can be in particular seen in Fig. 4 and Fig. 5, a wire 154 is directly connected to a (radial outer) wire attachment portion 156 of the wire wheel 146. The wire 154 runs into a wire pipe 158, which is attached to, e.g. glued to, the handle housing 104 and which guides the wire 154 in a proximal-distal direction out of the endoscope handle 100 into the (not shown) insertion cord 112, where it is guided towards the distal tip unit 118. In the distal tip unit 118 the wire 154 is fixed to an elevator element 160 formed as an Albarran lever, as e.g. shown in the explanatory view of Fig. 3 relating to the related art endoscope 2. The elevator element 160 can be brought in a first lowered elevator position 162 and a second raised elevator position 164 as schematically illustrated in Fig. 13 and 14. When the manually operable element 134 is rotated from the first operation position 150 shown in Fig. 4 to the second operation position 152 shown in Fig. 5 by a user/ operator, said manual operation is transmitted via elevator mechanics 166 - consisting of the operation wheel 140 and the wire wheel 146 - and the wire 154 to the elevator element 160 such that the first operation position 150 of the manually operable element 134 corresponds to the first lowered elevator position 162 and the second operation position 152 of the manually operable element 134 corresponds to the second raised elevator position 164. Said differently, a distal or downward rotation of the manually operable element 134 leads to an upward movement of the elevator element 160, and a proximal or upward rotation of the manually operable element 134 leads to a downward movement of the elevator element 160. This is reached since the operation wheel 140 and the wire wheel 146 rotate in different directions, or said differently since a motion inversion is provided by the elevator mechanics 166 according to the present disclosure. By moving the elevator element 160 between the first lowered elevator position 162 and the second raised elevator position 164, it is possible set an angle with respect to a longitudinal axis (proximal-distal-direction) of the distal tip unit 118 at which a tool or instrument coming from a (not shown) working channel 168 of the endoscope 102 is inserted into the patient's body cavity.

In Fig. 7 a first operation unit 170 and a second operation unit 172 are shown, which are both formed as control wheels. The operation units 170, 172 are arranged concentrically and coaxially with respect to each other and are both provided outside the handle housing 104 so as to be accessible to a user/ operator, in particular on one of the first and second side surfaces/ walls 126. Each of the operation units 170, 172 is connected to a (not shown) shaft, and each of the shafts is connected to a wire drum 174, in which a steering wire/ steering wires for bending the bending section 116 of the insertion cord 112 run. It is evident from Fig. 4, Fig. 5, Fig. 6 and Fig. 7 that the operation wheel 140 is coaxially and concentrically arranged to the operation units 170, 172, the shafts and the wire drums 174. In particular, there is provided an opening in the respective side surface/ wall 126 in combination with a hollow cylindrical protrusion 176 extending into the handle housing 104, wherein the operation wheel 140 is arranged/ provided/ supported on an outer side of the hollow cylindrical protrusion 176 inside the handle housing 104 and the shafts are supported on an inner side of the opening/ the hollow cylindrical protrusion 176. As is evident from Fig. 7, the manually operable element 134 and the operation units 170, 172 are preferably spaced apart, as the operation units 170, 172 are provided on the respective side surface/ wall 126 and the manually operable element 134 is provided in the rounded transition area 130 between the front surface/ wall 128 and the bottom surface/ wall 124, such that the manually operable element 134 and the operation units 170, 172 do not mutually affect each other. It is added that the operation units 170, 172, the wire drum 174 and further operation parts are not shown in Fig. 6 in order to better illustrate the elevator mechanics 166.

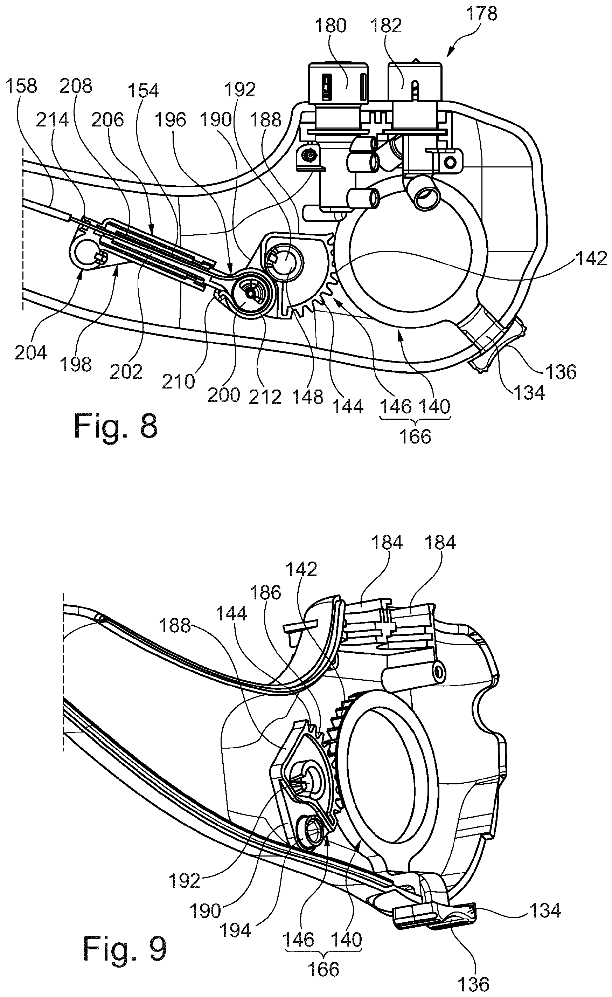

Due to the concentric and coaxial arrangement of the operations units 170, 172, the shafts, the wire drums 174 and the operation wheel 140, and due to the arrangement of the wire wheel 146 distally with respect to the operation wheel 140 and in close proximity to the bottom surface/ wall 124, there is much space in the proximal handle housing portion 108, in particular above the operation wheel 140 and the wire wheel 146 for other components like a valve assembly 178 having a gas/ water injection valve 180 and a suction valve 182 as illustrated in Fig. 8. As can be in particular seen in Fig. 7, the handle housing 104 comprises valve accommodation openings 184, which are provided for accommodating the gas/ water injection valve 180 and the suction valve 182.

As is evident when looking at Fig. 4 and Fig. 5 the wire 154 moves quite around inside the handle housing 104 when the wire wheel 146 rotates, which leads to the wire 154 being angled with respect to an orientation/ direction of the wire pipe 158, in particular when the manually operable element 134 is in the second operation position 152. In this light, a second preferred embodiment according to the present disclosure, which is shown in Fig. 8, Fig. 9, Fig. 10, Fig. 11, Fig. 12, Fig. 13 and Fig. 14, has been designed to overcome a problem of the wire 154 moving excessively around, potentially leading to a disconnection of the wire pipe 158 from the handle housing 104 or even to a kinking of the wire pipe 158.

With respect to the second preferred embodiment according to the present disclosure, essentially only differences compared to the first preferred embodiment are described. Apart from that, the explanations provided for the first preferred embodiment apply mutatis mutandis for the second preferred embodiment.

According to the second preferred embodiment it also applies that a manual operation/ rotation of the manually operable element 134 is transmitted via elevator mechanics 166 comprising an operation wheel 140 having an operation wheel gear portion 142 and a wire wheel 146 having a wire wheel gear portion 144 and a wire 154 to an elevator element 160 provided in the distal tip unit 118 of the insertion cord 112.

The manually operable element 134 has a concavely curved, ribbed gripping surface 136 according to the second preferred embodiment.

The wire wheel 146 of the second preferred embodiment has a circular portion 186 and two edges, namely a first edge 188 and a second edge 190, which are angled with respect to each other, in particular between 90° and 180°. The wire wheel 146 has thus a shape which corresponds to a cut-out portion or part of a disc and thus not a full disc as it is the case in the first preferred embodiment. The wire wheel 146 according to the second preferred embodiment comprises a rotational center 192 defined by an accommodation opening for the protrusion 148, the wire wheel gear portion 144 which is provided in the circular portion 186, and a hollow cylindrically formed bearing portion 194. The bearing portion 194 is provided radially away from the rotational center 192 of the wire wheel 146, in particular in a radially outer portion of the wire wheel 146. A rotation axis of the wire wheel 146 is parallel to an axis of the bearing portion 194.

According to the second preferred embodiment and as can be seen in particular in Fig. 8, Fig. 11, Fig. 12, Fig. 13 and Fig. 14, a connector part 196 is provided which is rotatably mounted on/ around the bearing portion 194 of the wire wheel 146. Further a guide rail part 198 is provided which is rotatably mounted in the handle housing 104 of the endoscope handle 100. The connector part 196 is slidingly accommodated in the guide rail part 198. In particular, the connector part 196 comprises a disc-shaped connector mounting portion 200 which is rotatably mounted on the bearing portion 194 of the wire wheel 146, and a rod-like slide portion 202. The guide rail part 198 comprises a rail mounting portion 204 rotatably mounted on (a protrusion of) the handle housing 104 and a guide rail portion 206 having a guide rail 208. The rod-like slide portion 202 of the connector part 196 is slidingly accommodated in the guide rail portion 206 of the guide rail part 198. The rail mounting portion 204 of the guide rail part 198 is provided at a distal portion of the guide rail part 198 and the guide rail portion 206 extends from the rail mounting portion 204 in a proximal direction towards the wire wheel 146. The wire 154 is fixed, e.g. by gluing, crimping, a screwed connection, a soldered connection, etc., to a fixation portion 210 of the connector mounting portion 200 of the connector part 196. The wire 154 is fixed to the connector mounting portion 200 of the connector part 196, forms a loop 212 in the connector mounting portion 200 of the connector part 196, and extends through the slide portion 202 of the connector part 196 and through a wire outlet opening 214 provided in the guide rail part 198. Since the rail mounting portion 204 of the guide rail part 198 is provided at the distal portion/ end of the guide rail part 198 adjacent the wire outlet opening 214, the wire outlet opening 214 remains at an essentially stationary/ fixed position throughout an operation range of the manually operable element 134, in other words experiences only little rotational displacement. A proximal end portion of the wire pipe 158 is arranged adjacent the wire outlet opening 214 of the guide rail part 198. An extension direction of the proximal end portion of the wire pipe 158 essentially corresponds to an axis of the guide rail portion 206 of the guide rail part 198 and thus to an axis of the rod-like slide portion 202 of the connector part 196, at least in any position of the manually operable element 134 between the first operation position 150 and the second operation position 152.

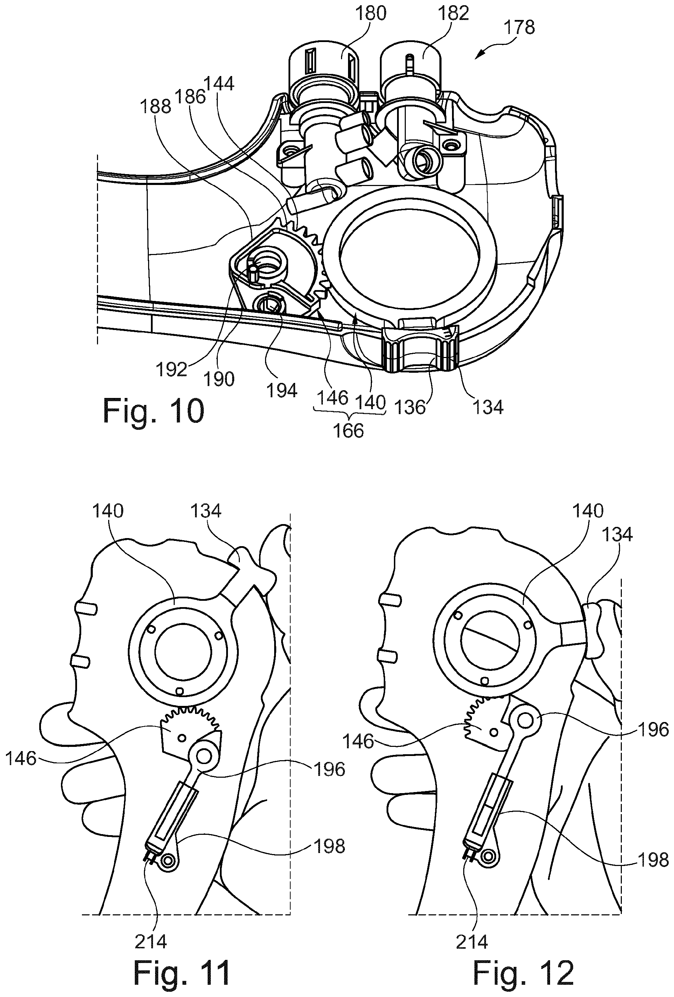

A formation of the wire wheel 146 according to the second preferred embodiment can best be seen in the perspective views of Fig. 9 and Fig. 10, in particular as the connector part 196 and the guide rail part 198 are not shown in Fig. 9 and Fig. 10. Fig. 9 shows valve accommodation openings 184 and Fig. 10 shows the valve assembly 178 comprising the gas/ water injection valve 180 and the suction valve 182 being assembled in the valve accommodation openings 184. Fig. 8 shows the connector part 196 being rotatably mounted to the wire wheel 146. It becomes clear that when the wire 154 is fixed to the connector part 196 and when the guide rail part 198 orients the connector part 196 as shown in Fig. 8, the wire 154 runs through the connector part 196 and directly into the wire pipe 158.

Fig. 11 shows the manually operable element 134 in its first operation positon 150, and Fig. 12 shows the manually operable element 134 in its second operation position 152. It becomes clear, that when moving the manually operable element 134 from the first operation position 150 to the second operation position 152, the connector part 196 is retracted in the guide rail part 198, so as to apply a pulling force on a wire 154 fixed to the connector part 196, which pulling force may serve to transfer an elevator element 160 provided in the distal tip unit 118 of the insertion cord 112 to a second raised elevator position 164. Moving in the other direction applies a pushing force on the wire.

Fig. 13 and Fig. 14 show an entire endoscope 102, with a half shell 106 of the handle housing 104 removed. In particular, also the working channel 168 of the endoscope 102 is shown, as well as the insertion tube 114, the bending section 116 and the distal tip unit 118 of the insertion cord 112. It becomes clear that when the manually operable element 134 is in its first operation position 150 shown in Fig. 13, an elevator element 160 formed as Albarran lever is in its first lowered elevator position 162, and when the manually operable element 134 is in its second operation position 152 shown in Fig. 14, the elevator element 160 is in its second raised elevator position 164. In Fig. 13 and Fig. 14 a monitor M is shown which may be connected to the endoscope 102.

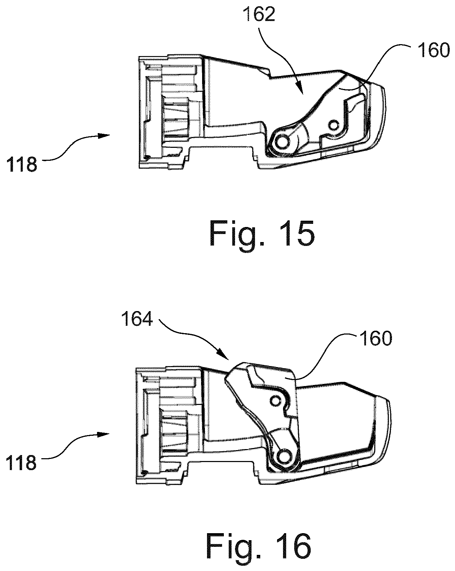

Fig. 15 shows a side view of the distal tip unit 118 of the endoscope 102 according to the present disclosure with the elevator element 160 formed as Albarran lever in the first lowered elevator position 162. Fig. 16 shows a side view of a distal tip unit 118 of the endoscope 102 according to the present disclosure with the elevator element 160 formed as Albarran lever in the second raised elevator position 164.

### List of reference signs

- 2: endoscope
- 4: endoscope handle
- 6: handle housing
- 8: manually operable element
- 10: elevator element
- 12: distal tip unit
- 14: operation wheel
- 16: slider rod
- 18: slider
- 20: wire
- 22: wire pipe
- 24: insertion cord
- 26: elevator mechanics
- 28: first operation position
- 29: first lowered elevator position
- 30: second operation position
- 31: second raised elevator position
- 32: rotational center
- 34: first slider rod connection point
- 36: second slider rod connection point

- 100: endoscope handle
- 102: endoscope
- 104: handle housing
- 106: half shell
- 108: proximal handle housing portion
- 110: distal handle housing portion
- 112: insertion cord
- 114: insertion tube
- 116: bending section
- 118: distal tip unit
- 120: receiving space
- 122: top surface/ wall
- 124: bottom surface/ wall
- 126: first/ second side surface/ wall
- 128: front surface/ wall
- 130: rounded transition area
- 132: transition region
- 134: manually operable element
- 136: gripping surface
- 138: slot-shaped opening
- 140: operation wheel
- 142: operation wheel gear portion
- 144: wire wheel gear portion
- 146: wire wheel
- 148: protrusion
- 150: first operation position
- 152: second operation position
- 154: wire
- 156: wire attachment portion
- 158: wire pipe
- 160: elevator element
- 162: first lowered elevator position
- 164: second raised elevator position
- 166: elevator mechanics
- 168: working channel
- 170: first operation unit
- 172: second operation unit
- 174: wire drum
- 176: hollow cylindrical protrusion
- 178: valve assembly
- 180: gas/ water injection valve
- 182: suction valve
- 184: valve accommodation opening
- 186: circular portion
- 188: first edge
- 190: second edge
- 192: rotational center
- 194: bearing portion
- 196: connector part
- 198: guide rail part
- 200: connector mounting portion
- 202: slide portion
- 204: rail mounting portion
- 206: guide rail portion
- 208: guide rail
- 210: fixation portion
- 212: loop
- 214: wire outlet opening

## Claims

1. An endoscope (102) comprising:
an endoscope handle (100) comprising a handle housing (104) and at least one manually operable element (134); and
an insertion cord (112) configured to be inserted into a patient's body cavity and comprising a distal tip unit (118), the distal tip unit (118) comprising an elevator element (160) movable between a first lowered elevator position (162) and a second raised elevator position (164) for changing a direction of a tool or an instrument inserted into the patient's body cavity via a working channel (168) of the endoscope (102); wherein
the manually operable element (134) is connected to the elevator element (160) via elevator mechanics (166) provided inside the handle housing (104) and a pull wire (154), and a manual operation of the manually operable element (134) is transmitted via the elevator mechanics (166) and the pull wire (154) to the elevator element (160) such that a first operation position (150) of the manually operable element (134) corresponds to the first lowered elevator position (162) and a second operation position (152) of the manually operable element (134) corresponds to the second raised elevator position (164); wherein
the elevator mechanics (166) comprise: an operation wheel (140) connected to the manually operable element (134) and comprising an operation wheel gear portion (142); and a wire wheel (146) comprising a wire wheel gear portion (144); wherein the operation wheel gear portion (142) is in meshing engagement with the wire wheel gear portion (144); wherein the pull wire (154) is connected to the wire wheel (146);
**characterised in that**:
the pull wire (154) is directly attached to a portion of the wire wheel (146) or is indirectly connected to the wire wheel (146) via a connector part (196) which is rotatably mounted on the wire wheel (146) and to which the pull wire (154) is fixedly attached.

2. Endoscope (102) according to claim 1, wherein the elevator mechanics (166) are configured such that
when the manually operable element (134) is moved or rotated in a distal direction from the first operation position (150) to the second operation position (152), the elevator element (160) is transferred from the first lowered elevator position (162) to the second raised elevator position (164); and
when the manually operable element (134) is moved or rotated in a proximal direction from the second operation position (152) to the first operation position (150), the elevator element (160) is transferred from the second raised elevator position (164) to the first lowered elevator position (162).

3. Endoscope (102) according to claim 1 or 2, wherein the operation wheel gear portion (142) of the operation wheel (140) and the wire wheel gear portion (144) of the wire wheel (146) are external gears comprising external teeth.

4. Endoscope (102) according to any one of claims 1 to 3, wherein a radius of the operation wheel gear portion (142) is greater than a radius of the wire wheel gear portion (144).

5. Endoscope (102) according to any one of claims 1 to 4, wherein the wire wheel (146) is arranged distally with respect to the operation wheel (140) inside the handle housing (104) of the endoscope handle (100).

6. Endoscope (102) according to any one of claim 1 to 5, wherein a wire pipe (158) is fixed to the handle housing (104), and the pull wire (154) runs into and is guided in the wire pipe (158).

7. Endoscope (102) according to any one of claims 1 to 6, wherein the operation wheel (140) is arranged coaxially with respect to an operation unit (170, 172) and a wire drum (174) provided for actively bending a bending section (116) of the insertion cord (112).

8. Endoscope (102) according to any one of claims 1 to 7, wherein the wire wheel (146) has a circular portion (186) and two edges (188, 190) angled with respect to each other such that the wire wheel (146) has a shape corresponding to a cut-out portion or part of a disc, the wire wheel (146) comprising at least a rotational center (192) and the wire wheel gear portion (144) provided in the circular portion (186).

9. Endoscope (102) according to any one of claims 1 to 8, wherein a guide rail part (198) is rotatably mounted in the handle housing (104), and the connector part (196) is slidingly accommodated in the guide rail part (198).

10. Endoscope (102) according to claim 9, wherein the connector part (196) and the guide rail part (198) are arranged distally with respect to the operation wheel (140) and the wire wheel (146).

11. Endoscope (102) according to claim 9 or 10, wherein
the connector part (196) comprises a disc-shaped connector mounting portion (200) configured to be rotatably mounted on the wire wheel (146), and a rod-like slide portion (202);
the guide rail part (198) comprises a distal rail mounting portion (204) configured to be rotatably mounted on the handle housing (104) and a guide rail portion (206) having a guide rail (208); and
the rod-like slide portion (202) of the connector part (196) is slidingly accommodated in the guide rail portion (206) of the guide rail part (198).

12. Endoscope (102) according to claim 11, wherein the pull wire (154) is looped through and fixed to the disc-shaped connector mounting portion (200) and extends through the rod-like slide portion (202).

13. Endoscope (102) according to any of claims 9 to 12, wherein a proximal end portion of a wire pipe (158) fixed to the handle housing (104) is arranged adjacent a distal end portion of the guide rail part (198).

14. System comprising: an endoscope (102) according to any one of the preceding claims 1 to 13; and a monitor (M).

## Patentansprüche

1. Endoskop (102) mit:
einem Endoskopgriff (100) mit einem Griffgehäuse (104) und mindestens einem manuell betätigbaren Element (134); und
einem Einführungsstrang (112), der zum Einführen in die Körperhöhle eines Patienten eingerichtet ist und eine distale Spitzeneinheit (118) umfasst, wobei die distale Spitzeneinheit (118) ein Hebeelement (160) umfasst, das zwischen einer ersten abgesenkten Hebeposition (162) und einer zweiten angehobenen Hebeposition (164) bewegbar ist zur Änderung der Richtung eines Werkzeugs oder Instruments, das über einen Arbeitskanal (168) des Endoskops (102) in die Körperhöhle des Patienten eingeführt ist; wobei
das manuell betätigbare Element (134) über eine im Griffgehäuse (104) vorgesehene Hebemechanik (166) und einen Zugdraht (154) mit dem Hebeelement (160) verbunden ist und eine manuelle Betätigung des manuell betätigbaren Elements (134) über die Hebemechanik (166) und den Zugdraht (154) auf das Hebeelement (160) übertragen wird, so dass eine erste Betätigungsposition (150) des manuell betätigbaren Elements (134) der ersten abgesenkten Hebeposition (162) entspricht und eine zweite Betätigungsposition (152) des manuell betätigbaren Elements (134) der zweiten angehobenen Hebeposition (164) entspricht; wobei
die Hebemechanik (166) umfasst: ein Betätigungsrad (140), das mit dem manuell betätigbaren Element (134) verbunden ist und einen Betätigungsrad-Zahnradabschnitt (142) umfasst; und ein Drahtrad (146), das einen Drahtrad-Zahnradabschnitt (144) umfasst; wobei der Betätigungsrad-Zahnradabschnitt (142) mit dem Drahtrad-Zahnradabschnitt (144) in Eingriff steht; wobei der Zugdraht (154) mit dem Drahtrad (146) verbunden ist; **dadurch gekennzeichnet, dass**
der Zugdraht (154) direkt an einem Abschnitt des Drahtrades (146) befestigt ist oder indirekt über ein Verbindungsteil (196) mit dem Drahtrad (146) verbunden ist, das drehbar am Drahtrad (146) angebracht ist und an dem der Zugdraht (154) fest befestigt ist.

2. Endoskop (102) gemäß Anspruch 1, wobei die Hebemechanik (166) so konfiguriert ist, dass
wenn das manuell betätigbare Element (134) in distaler Richtung von der ersten Betätigungsposition (150) zur zweiten Betätigungsposition (152) bewegt oder gedreht wird, das Hebeelement (160) von der ersten abgesenkten Hebeposition (162) in die zweite angehobene Hebeposition (164) gebracht wird; und
wenn das manuell betätigbare Element (134) in proximaler Richtung von der zweiten Betätigungsposition (152) zur ersten Betätigungsposition (150) bewegt oder gedreht wird, das Hebeelement (160) von der zweiten angehobenen Hebeposition (164) in die erste abgesenkte Hebeposition (162) gebracht wird.

3. Endoskop (102) nach Anspruch 1 oder 2, wobei der Betätigungsrad-Zahnradabschnitt (142) des Betätigungsrades (140) und der Drahtrad-Zahnradabschnitt (144) des Drahtrades (146) Außenzahnräder mit Außenverzahnungen sind.

4. Endoskop (102) nach einem der Ansprüche 1 bis 3, wobei ein Radius des Betätigungsrad-Zahnradabschnitts (142) größer ist als ein Radius des Drahtrad-Zahnradabschnitts (144).

5. Endoskop (102) nach einem der Ansprüche 1 bis 4, wobei das Drahtrad (146) distal zum Betätigungsrad (140) innerhalb des Griffgehäuses (104) des Endoskopgriffs (100) angeordnet ist.

6. Endoskop (102) nach einem der Ansprüche 1 bis 5, wobei ein Drahtrohr (158) am Griffgehäuse (104) befestigt ist und der Zugdraht (154) in das Drahtrohr (158) hineinläuft und darin geführt wird.

7. Endoskop (102) nach einem der Ansprüche 1 bis 6, wobei das Betätigungsrad (140) koaxial zu einer Bedieneinheit (170, 172) und einer Drahttrommel (174) angeordnet ist, die zum aktiven Biegen eines Biegeabschnitts (116) des Einführungsstrangs (112) vorgesehen ist.

8. Endoskop (102) nach einem der Ansprüche 1 bis 7, wobei das Drahtrad (146) einen kreisförmigen Abschnitt (186) und zwei Kanten (188, 190) aufweist, die so zueinander abgewinkelt sind, dass das Drahtrad (146) eine Form aufweist, die einem ausgeschnittenen Abschnitt oder Teil einer Scheibe entspricht, wobei das Drahtrad (146) mindestens einen Drehpunkt (192) und den im kreisförmigen Abschnitt (186) vorgesehenen Drahtrad-Zahnradabschnitt (144) umfasst.

9. Endoskop (102) gemäß einem der Ansprüche 1 bis 8, wobei ein Führungsschienenteil (198) drehbar im Griffgehäuse (104) angebracht ist und das Verbindungsteil (196) gleitend im Führungsschienenteil (198) aufgenommen ist.

10. Endoskop (102) gemäß Anspruch 9, wobei das Verbindungsteil (196) und das Führungsschienenteil (198) distal zum Betätigungsrad (140) und zum Drahtrad (146) angeordnet sind.

11. Endoskop (102) gemäß Anspruch 9 oder 10, wobei
das Verbindungsteil (196) einen scheibenförmigen Verbindungsbefestigungsabschnitt (200), der so konfiguriert ist, dass er drehbar an dem Drahtrad (146) befestigt werden kann, und einen stabförmigen Gleitabschnitt (202) aufweist;
wobei das Führungsschienenteil (198) einen distalen Schienenbefestigungsabschnitt (204), der so konfiguriert ist, dass er drehbar an dem Griffgehäuse (104) befestigt werden kann, und einen Führungsschienenabschnitt (206) mit einer Führungsschiene (208) aufweist; und
der stabförmige Gleitabschnitt (202) des Verbindungsteils (196) gleitend in dem Führungsschienenabschnitt (206) des Führungsschienenteils (198) aufgenommen ist.

12. Endoskop (102) gemäß Anspruch 11, wobei der Zugdraht (154) durch den scheibenförmigen Verbindungsbefestigungsabschnitt (200) geschlungen und an diesem befestigt ist und sich durch den stabförmigen Gleitabschnitt (202) erstreckt.

13. Endoskop (102) gemäß einem der Ansprüche 9 bis 12, wobei ein proximaler Endabschnitt eines am Griffgehäuse (104) befestigten Drahtrohrs (158) benachbart zu einem distalen Endabschnitt des Führungsschienenteils (198) angeordnet ist.

14. System mit: einem Endoskop (102) gemäß einem der vorstehenden Ansprüche 1 bis 13; und einem Monitor (M).

## Revendications

1. Endoscope (102) comprenant :
une poignée d'endoscope (100) comprenant un boîtier de poignée (104) et au moins un élément actionnable manuellement (134) ; et
un cordon d'insertion (112) conçu pour être inséré dans une cavité corporelle d'un patient et comprenant une unité d'embout distal (118), l'unité d'embout distal (118) comprenant un élément élévateur (160) mobile entre une première position d'élévateur abaissée (162) et une seconde position d'élévateur élevée (164) permettant de modifier la direction d'un outil ou d'un instrument inséré dans la cavité corporelle du patient par le biais d'un canal de travail (168) de l'endoscope (102) ; dans lequel
l'élément actionnable manuellement (134) est relié à l'élément élévateur (160) par le biais d'un mécanisme élévateur (166) prévu à l'intérieur du boîtier de poignée (104) et d'un câble de traction (154), et l'actionnement manuel de l'élément actionnable manuellement (134) est transmise par le biais du mécanisme élévateur (166) et du câble de traction (154) à l'élément élévateur (160), de sorte qu'une première position de fonctionnement (150) de l'élément actionnable manuellement (134) corresponde à la première position d'élévateur abaissée (162) et qu'une seconde position de fonctionnement (152) de l'élément actionnable manuellement (134) corresponde à la seconde position d'élévateur élevée (164) ; dans lequel
le mécanisme élévateur (166) comprend : une roue d'actionnement (140) reliée à l'élément actionnable manuellement (134) et comprenant une partie d'engrenage de roue d'actionnement (142) ; et une roue à fil (146) comprenant une denture de roue à fil (144) ; la partie d'engrenage de roue d'actionnement (142) étant mise en prise par engrènement avec la partie d'engrenage de roue à fil (144) ; dans lequel le câble de traction (154) est relié à la roue à fil (146) ; **caractérisé en ce que** :
le câble de traction (154) est directement fixé à une partie de la roue à fil (146) ou est indirectement relié à la roue à fil (146) par le biais d'une pièce de connexion (196) qui est montée de manière rotative sur la roue à fil (146) et à laquelle le câble de traction (154) est fixé de manière fixe.

2. Endoscope (102) selon la revendication 1, dans lequel les mécanismes élévateurs (166) sont configurés de telle sorte que
lorsque l'élément actionnable manuellement (134) est déplacé ou pivoté dans une direction distale de la première position de fonctionnement (150) à la seconde position de fonctionnement (152), l'élément élévateur (160) est transféré de la première position d'élévateur abaissée (162) à la seconde position d'élévateur élevée (164) ; et
lorsque l'élément actionnable manuellement (134) est déplacé ou pivoté dans une direction proximale de la seconde position de fonctionnement (152) à la première position de fonctionnement (150), l'élément élévateur (160) est transféré de la seconde position d'élévateur élevée (164) à la première position d'élévateur abaissée (162).

3. Endoscope (102) selon la revendication 1 ou 2, dans lequel la partie d'engrenage de roue d'actionnement (142) de la roue d'actionnement (140) et la partie d'engrenage de roue à fil (144) de la roue à fil (146) sont des engrenages externes comprenant des dents externes.

4. Endoscope (102) selon l'une quelconque des revendications 1 à 3, dans lequel le rayon de la partie d'engrenage de roue d'actionnement (142) est supérieur à un rayon de la partie d'engrenage de roue à fil (144).

5. Endoscope (102) selon l'une quelconque des revendications 1 à 4, dans lequel la roue à fil (146) est agencée distalement par rapport à la roue d'actionnement (140) à l'intérieur du boîtier de poignée (104) de la poignée d'endoscope (100).

6. Endoscope (102) selon l'une quelconque des revendications 1 à 5, dans lequel un tube de fil (158) est fixé au boîtier de poignée (104), et le câble de traction (154) entre et est guidé dans le tube de fil (158).

7. Endoscope (102) selon l'une quelconque des revendications 1 à 6, dans lequel la roue d'actionnement (140) est agencée coaxialement par rapport à une unité d'actionnement (170, 172) et à un tambour à câble (174) prévu pour plier activement une section de pliage (116) du cordon d'insertion (112).

8. Endoscope (102) selon l'une quelconque des revendications 1 à 7, dans lequel la roue à fil (146) présente une partie circulaire (186) et deux bords (188, 190) inclinés l'un par rapport à l'autre de telle sorte que la roue à fil (146) présente une forme correspondant à une partie découpée ou à une partie d'un disque, la roue à fil (146) comprenant au moins un centre de rotation (192) et la partie d'engrenage de roue à fil (144) prévue dans la partie circulaire (186).

9. Endoscope (102) selon l'une quelconque des revendications 1 à 8, dans lequel une partie de rail de guidage (198) est montée de manière rotative dans le boîtier de poignée (104), et la pièce de connexion (196) est logée de manière coulissante dans la partie de rail de guidage (198).

10. Endoscope (102) selon la revendication 9, dans lequel la pièce de connexion (196) et la partie rail de guidage (198) sont agencées distalement par rapport à la roue d'actionnement (140) et à la roue à fil (146).

11. Endoscope (102) selon la revendication 9 ou 10, dans lequel
la pièce de connexion (196) comprend une partie de montage de connecteur en forme de disque (200) configurée pour être montée de manière rotative sur la roue à fil (146), et une partie coulissante en forme de tige (202) ;
la partie de rail de guidage (198) comprend une partie de montage de rail distal (204) configurée pour être montée de manière rotative sur le boîtier de poignée (104) et une partie de rail de guidage (206) présentant un rail de guidage (208) ; et
la partie coulissante en forme de tige (202) de la pièce de connexion (196) est logée de manière coulissante dans la partie rail de guidage (206) de la partie rail de guidage (198).

12. Endoscope (102) selon la revendication 11, dans lequel le câble de traction (154) est bouclé et fixé à la partie de montage de connecteur en forme de disque (200) et s'étend à travers la partie coulissante en forme de tige (202).

13. Endoscope (102) selon l'une quelconque des revendications 9 à 12, dans lequel une partie d'extrémité proximale d'un tube de fil (158) fixé au boîtier de poignée (104) est agencée de manière adjacente à une partie d'extrémité distale de la partie de rail de guidage (198).

14. Système comprenant : un endoscope (102) selon l'une quelconque des revendications précédentes 1 à 13 ; et un moniteur (M).
